# EUROPÄISCHE PATENTSCHRIFT

(11) **EP 1 171 092 B1**
(45) Veröffentlichungstag und Bekanntmachung des Hinweises auf die Patenterteilung: **08.01.2003**
(21) Anmeldenummer: 00926861.6
(22) Anmeldetag: 11.04.2000
(51) Int. Cl.: A61K 7/48

(54) **DEKORATIVE KOSMETISCHE ZUBEREITUNGEN**
DECORATIVE COSMETIC PREPARATIONS
PREPARATIONS COSMETIQUES DE MAQUILLAGE

(30) Priorität: 20.04.1999 DE 19917744
(43) Veröffentlichungstag der Anmeldung: 16.01.2002
(73) Patentinhaber: Biotec Pharmacon ASA, 9008 Tromsö (NO)
(72) Erfinder: WACHTER, Rolf, D-40595 Düsseldorf (DE); GRIESBACH, Ute, D-40597 Düsseldorf (DE); ANSMANN, Achim, D-40699 Erkrath (DE); FABRY, Bernd, D-41352 Korschenbroich (DE); ENGSTAD, Rolf, E., N-9008 Tromsö (NO)
(74) Vertreter: Melzer, Wolfgang, Dipl.-Ing.
(86) Internationale Anmeldenummer: EP0003193
(87) Internationale Veröffentlichungsnummer: WO00062754

(56) Entgegenhaltungen:
- WO-A-96/28476
- US-A- 3 659 025
- US-A- 5 158 772
- US-A- 5 223 491
- US-A- 5 385 832
- US-A- 5 653 967
- PATENT ABSTRACTS OF JAPAN vol. 015, no. 403 (C-0875), 15. Oktober 1991 (1991-10-15) & JP 03 167102 A (HARIMA CHEM INC), 19. Juli 1991 (1991-07-19)

## Beschreibung

### Gebiet der Erfindung

Die Erfindung befindet sich auf dem Gebiet der dekorativen Kosmetik und betrifft Zubereitungen, die ausgewählte Polysaccharide vom Typ der β-1,3-Glucane enthalten.

### Stand der Technik

Die altersbedingte Faltenbildung wird durch den Abbau von verschiedenen Makromolekülen wie beispielsweise Elastin und Kollagen hervorgerufen, für den Elastasen verantwortlich sind. Auch eine Vielzahl von entzündlichen Hauterkrankungen, wie beispielsweise Schuppenflechte oder UV-Erytheme, lassen sich ursächlich mit einer erhöhten Konzentration an Serinproteasen, wie z.B. Elastase in den oberen Hautschichten in Verbindung bringen [vgl. R.Voegeli et al. in **Cosm.Toil. 111, 51 (1996)].**

Der Faltenbildung in der Haut wird in der Regel nicht durch physiologisch wirksame Prinzipien, sondern durch kosmetische Mittel entgegengewirkt. Neben reinen "Antiaging Produkten" haben sich inzwischen im Markt auch eine Vielzahl von dekorativen Kosmetikprodukten etabliert, die zusätzlich eine pflegende und schützende Wirkung ausloben. Hierbei handelt es sich in der Regel um mit Wasser oder wäßrigen Wirkstoffen beladene Liposomen, die durch die Fettschicht der Haut in die Epidermis gelangen, sich dort allmählich auflösen und durch die kontinuierliche Wasserabgabe die Hautvertiefungen ausgleichen und den Feuchtigkeitsgehalt der Haut regulieren. Dieser Effekt stellt jedoch keine Bekämpfung der Ursachen dar, sondern hat lediglich einen sogenannten "repair effect", der zudem nur über eine kurze Zeit andauert.

Die Verwendung von speziellen Polysacchariden als Mittel gegen die Hautalterung ist aus dem Stand der Technik bekannt. So wird beispielsweise in der Patentschrift **US 5,223,491** vorgeschlagen, ein carboxymethyliertes β-1,3-Glucan, das aus dem Hefepilz *Saccharomyces cerewsiae* extrahiert wurde, für die topische Anwendung einzusetzen. Das Glucan ist jedoch wasserunlöslich und kann daher nur mit großen Schwierigkeiten formuliert werden. Gemäß der Lehre der beiden Druckschriften **DE-A1 3744345** (Lomapharm) und **EP-B1 0175667** (Larm) eignen sich Glucane zur Stimulation der Makrophagenaktivität. Die pharmazeutische Wirkung verschiedener Glucane ist des weiteren aus den beiden Europäischen Patentanmeldungen **EP-A1 045338** (Debat) und **EP-A1 0561408** (Kaken) bekannt. Gegenstand der Europäischen Patentschrift **EP-B1 0500718** (Donzis) ist der Einsatz von wasserunlöslichen β-(1,3)-Glucanen, die aus den Zellwänden von Hefen gewonnen werden, zur Revitalisierung der Haut.

Dekorative Kosmetische Zubereitungen enthaltend wasserlösliche β-(1,3)-glucane die im wesentlichen frei von β(1,6)-Verknüpfungen sind im WO 00/54743 und im WO 00 547 41 beschrieben.

Die komplexe Aufgabe der Erfindung hat demnach darin bestanden, dekorative kosmetische Zubereitungen, wie z.B. Make-Ups, Lippenstifte, Lidschatten etc. zur Verfügung zu stellen, die neben der von dem von Verbraucherin gewünschten farblich akzentuierenden Effekt eine dem Verlust von Feuchtigkeit und der Faltenbildung vorbeugende sowie gleichzeitig pflegende und immunstimulierende Wirkung aufweisen.

### Beschreibung der Erfindung

Gegenstand der Erfindung sind dekorative kosmetische Zubereitungen, enthaltend wasserlösliche β-(1,3)-Glucane, die im wesentlichen frei von β-(1,6)-Verknüpfungen sind.

Überraschenderweise wurde gefunden, daß dekorative kosmetische Zubereitungen, die im Sinne der Erfindung die speziellen Glucane enthalten, gegenüber den Produkten des Stands der Technik eine verbesserte Pflegeleistung besitzen. Gleichzeitig wird die Feuchtigkeitsbindung erhöht, der Faltenbildung entgegengewirkt und das Immunsystem stimuliert. Unter dekorativen kosmetischen Zubereitungen werden in diesem Zusammenhang tönende Gesichtscremes, Make-Up's, Rouges, Gesichtspuder, Lidschatten, Kajale, Wimpemtuschen (Mascaras), Eyeliner, Lippenstifte, Abdeckstifte, Nagellacke und dergleichen verstanden.

### Wasserlösliche β-(1,3)-Glucane

Unter der Bezeichnung Glucane werden Homopolysaccharide auf Basis der Glucose verstanden. Je nach sterischer Verknüpfung unterscheidet man zwischen β-(1,3)-, β-(1,4)- und β-(1,6)-Glucanen. β-(1,3)-Glucane weisen meist eine helicale Struktur auf, während Glucane mit einer 1,4-Verknüpfung im allgemeinen eine lineare Struktur besitzen. Die β-Glucane der Erfindung besitzen eine (1,3)-Struktur, d.h. sie sind weitgehend frei von unerwünschten (1,6)-Verknüpfungen. Vorzugsweise werden solche β-(1,3)-Glucane eingesetzt, deren Seitenketten ausschließlich (1,3)-Verknüpfungen aufweisen. Insbesondere enthalten die Mittel Glucane, die auf Basis von Hefen der Familie *Saccharomyces*, speziell *Sac charomyces cerevisiae* erhalten werden. Glucane dieser Art sind in technischem Maße nach den Verfahren des Stands der Technik zugänglich. So beschreibt die Internationale Patentanmeldung **WO 95/30022** (Biotec-Mackzymal) ein Verfahre zur Herstellung solcher Stoffe, bei dem man Glucane mit β-(1,3)- und β-(1,6)-Verknüpfungen in solcher Weise mit β-(1,6)-Glucanasen in Kontakt bringt, daß praktisch alle β-(1,6)-Verknüpfungen gelöst werden. Vorzugsweise werden zur Herstellung der Glucane Glucänasen auf Basis von *Trichodermia harzianum* eingesetzt. Soweit es die Herstellung und Zugänglichkeit der in den erfindungsgemäßen Mitteln enthaltenen Glucane angeht, wird in vollem Umfang auf die Offenbarung der oben genannten Schrift Bezug genommen. Die Glucane können in den Zubereitungen in Mengen von 0,1 bis 5, vorzugsweise 0,2 bis 5 und insbesondere 0,5 bis 1 Gew.-% - bezogen auf die Mittel - enthalten sein.

### Gewerbliche Anwendbarkeit

Die erfindungsgemäßen Zubereitungen können femer als weitere Hilfs- und Zusatzstoffe Ölkörper, Emulgatoren, Überfettungsmittel, Konsistenzgeber, Verdickungsmittel, Polymere, Siliconverbindungen, Fette, Wachse, Stabilisatoren, biogene Wirkstoffe, Deowirkstoffe, Quellmittel, UV-Lichtschutzfaktoren, Antioxidantien, organische wie anorganische Farbpigmente, Hydrotrope, Konservierungsmittel, Solubilisatoren, Parfümöle, Farbstoffe und dergleichen enthalten. Die Zubereitungen können wasserfrei oder praktisch wasserfrei sein (z.B. Gesichtspuder oder Nagellack), sie können jedoch Wasser im Sinne eines weiteren Hilfsstoffes auch in größeren Mengen enthalten (z.B. cremeförmige Zubereitungen). Vorzugsweise enthalten die erfindungsgemäßen Zubereitungen
(a) 0,1 bis 1 Gew.-% wasserlösliche β-(1,3)-Glucane, die im wesentlichen frei von β-(1,6)-Verknüpfungen sind, und
(b) 99 bis 99,9 Gew.-% Hilfs- und Zusatzstoffe.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen-und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar, Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840** A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, VinylpyrrolidonNinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben, UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester; 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP-A1 0818450** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Hierbei zeichnen sich Kombinationen von Octocrylene bzw. Campherderivaten mit Butyl Methoxydibenzoylmethane durch besondere Photostabilität aus.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfaktoren sind der Übersicht von P.Finkel in **SÖFW-Journal 122, 543 (1996)** zu entnehmen.

Neben den primären Lichtschutzstoffen können auch sekundäre Lichtschutzmittel vom Typ der **Anti oxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesterylund Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B.

Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können femer **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedern-, Rosenholz), Krautern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe.

Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Mehylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 95 bis 99,9, vorzugsweise 99 bis 99,5 Gew.-% - bezogen auf die Mittel - betragen.

### Beispiele

Die nachfolgende Tabelle enthält eine Reihe von Formulierungsbeispiele für verschiedene dekorative Kosmetikprodukte unter Einsatz von Betaglucan (Highcareen® GS, Henkel KGaA, Düsseldorf/FRG). Alle Angaben verstehen sich als Gew.-%. Es bedeuten: (1) Getönte Tagescreme; (2) Pudercreme; (3) Gepreßter Gesichtspuder; (4) Loser Gesichtspuder; (5) Rouge; (6) Lip Gloss; (7) Pflegender Lippenstift; (8) Dekorativer Lippenstift; (9) Eyeliner; (10) Wimpemtusche; (11) Gepreßter Lidschatten; (12) Lidschatten in Emulsionsform; (13) Pearllack; (14) Cremelack.

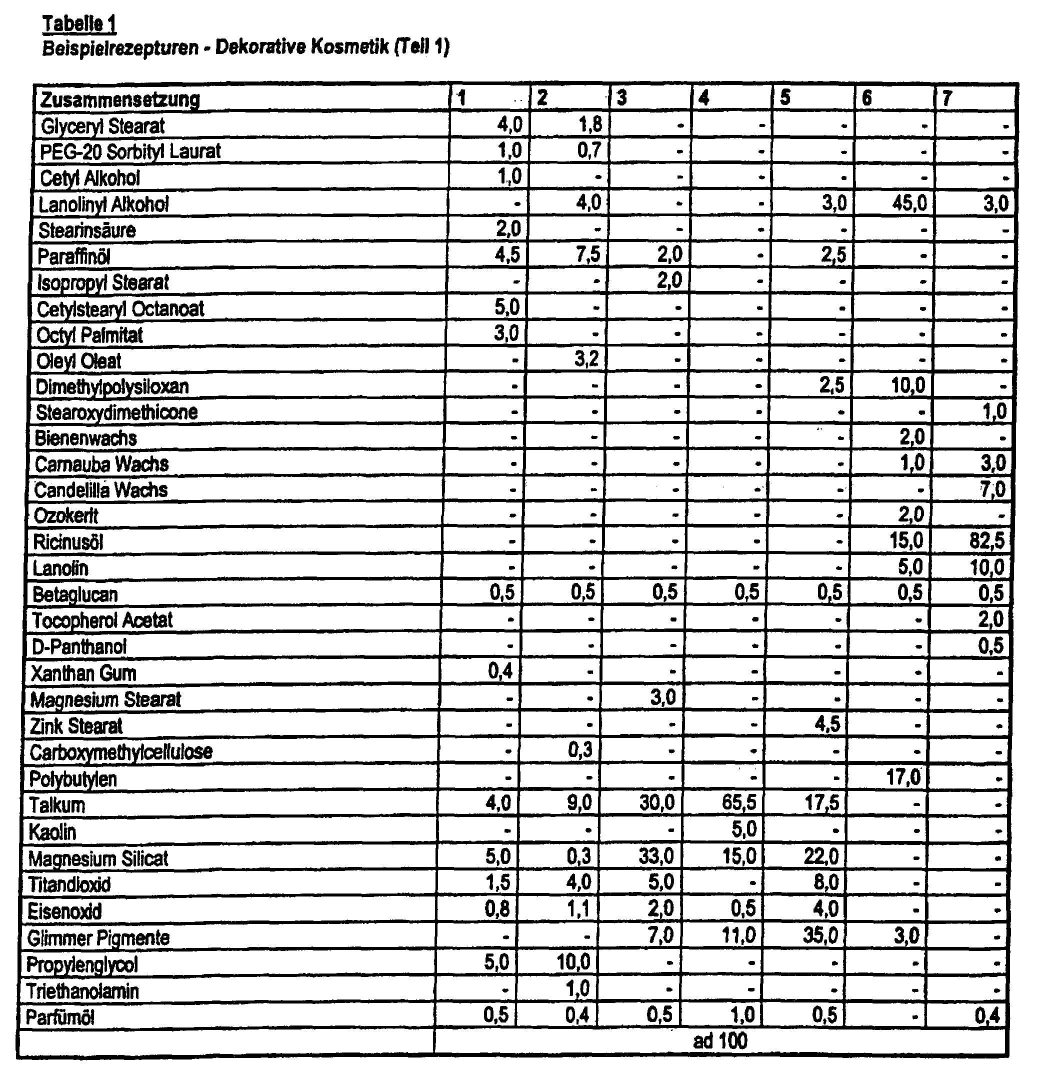

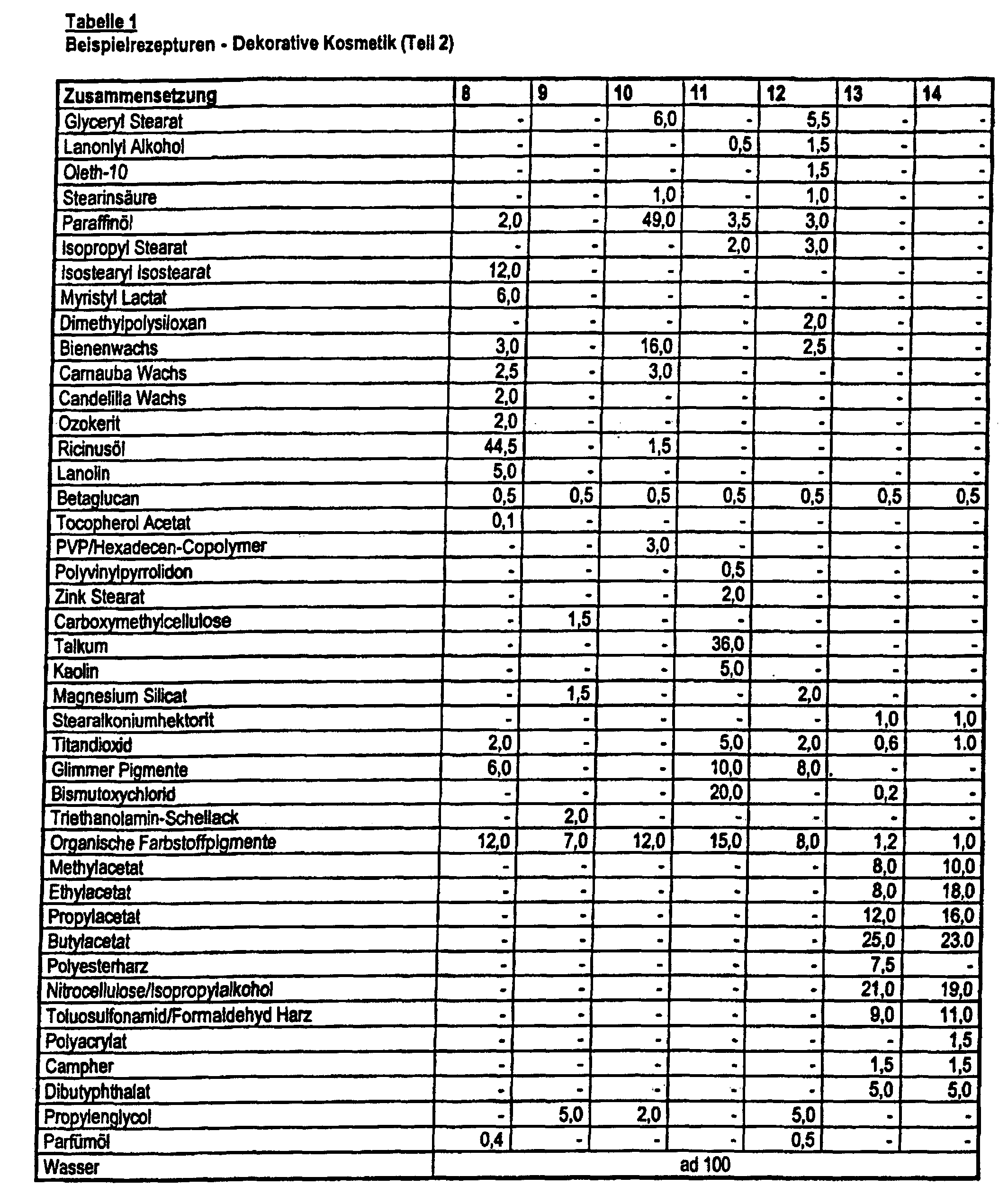

## Patentansprüche

1. Dekorative kosmetische Zusammensetzung, enthaltend wasserlösliche β-(1,3)-Glucane, die im wesentlichen frei von β-(1,6)-Verknüpfungen sind, **dadurch gekennzeichnet, dass**
die wasserlöslichen β-(1,3)-Glucane nicht als Nanoteilchen vorliegen, und
die Zusammensetzung frei von (Desoxy)Ribonukleinsäuren sowie deren Spalt- und Abbauprodukte ist.

2. Zusammensetzungen nach Anspruch 1, ausgewählt aus der Gruppe aus tönenden Gesichtscremes, Make'ups, Rouges, Gesichtspuder, Lidschatten, Kajale, Wimperntusche (Maskaras), Eyeliner, Lippenstifte, Abdeckstifte, Nagellacke und dergleichen.

3. Zubereitung nach Anspruch 1 oder 2, **dadurch gekennzeichnet, dass** sie Glucane enthält, die auf Basis von Hefen der Familie *Saccharomyces* erhalten werden.

4. Zubereitung nach Anspruch 1, 2 oder 3, **dadurch gekennzeichnet, dass** sie Glucane enthält, die erhalten werden, indem man Glucane mit β-(1,3)- und β-(1,6)-Verknüpfungen in solcher Weise mit β-(1,6)-Glucanasen in Kontakt bringt, dass praktisch alle β-(1,6)-Verknüpfungen gelöst werden.

5. Zubereitung nach einem der vorstehenden Ansprüche, **dadurch gekennzeichnet, dass** sie Glucane enthält, die zuvor mit Glucanasen auf Basis von *Trichodermia harzianum* behandelt worden sind.

6. Verwendung von wasserlöslichen β-(1,3)-Glucanen, die im wesentlichen frei von β-(1,6)-Verknüpfungen sind, zur Herstellung von dekorativen kosmetischen Zusammensetzungen nach einem der vorstehenden Ansprüche.

## Claims

1. Decorative cosmetic composition comprising water-soluble β-(1,3)-glucans which are essentially free from β-(1,6) linkages, **characterized in that**
the water-soluble β-(1,3)-glucans are not in the form of nanoparticles, and
the composition is free from (deoxy)ribonucleic acids and their cleavage and degradation products.

2. Compositions according to Claim 1, chosen from the group of tinted face creams, foundations, blushers, face powders, eyeshadows, kohl pencils, mascaras, eyeliners, lipsticks, concealing sticks, nail varnishes and the like.

3. Preparation according to Claim 1 or 2, **characterized in that** it comprises glucans obtained on the basis of yeasts from the *Saccharomyces* family.

4. Preparation according to Claim 1, 2 or 3, **characterized in that** it comprises glucans which are obtained by bringing glucans with β-(1,3) and β-(1,6) linkages into contact with β-(1,6)-glucanases in such a way that virtually all of the β-(1,6) linkages are broken.

5. Preparation according to one of the preceding claims, **characterized in that** it comprises glucans which have been treated beforehand with glucanases based on *Trichodermia harzianum*.

6. Use of water-soluble β-(1,3)-glucans which are essentially free from β-(1,6) linkages for the preparation of decorative cosmetic compositions according to one of the preceding claims.

## Revendications

1. Composition cosmétique pour le maquillage contenant des β-(1,3)-glucanes solubles dans l'eau, lesquels sont pratiquement exempts de liaisons β-(1,6), **caractérisée en ce que**
les β-(1,3)-glucanes solubles dans l'eau ne sont pas présents en tant que nanoparticules, et
la composition est exempte d'acides (désoxy)ribonucléiques ainsi que de leurs produits de dissociation et de décomposition.

2. Compositions selon la revendication 1 choisies parmi des crèmes teintées pour le visage, des maquillages, des rouges à lèvres, des poudres pour le visage, des ombres à paupières, des kajals, des crèmes pour cils (mascaras), des eyeliners, des crayons à lèvres, des crayons correcteurs, des vernis à ongles et semblables.

3. Préparations selon la revendication 1 ou 2, **caractérisées en ce qu'**elles contiennent des glucanes qui sont obtenus à partir de levures de la famille *Saccharomyces*.

4. Préparations selon la revendication 1, 2 ou 3, **caractérisées en ce qu'**elles contiennent des glucanes qui sont obtenus **en ce que** l'on met des glucanes avec des liaisons β-(1,3) et β-(1,6) en contact avec des β-(1,6)-glucanases de telle sorte que pratiquement toutes les liaisons β-(1,6) sont dissoutes.

5. Préparations selon l'une quelconque des revendications précédentes **caractérisées en ce qu'**elles contiennent des glucanes qui ont été préalablement traités avec des glucanases à base de *Trichodermia harzianum*.

6. Utilisation de β-(1,3)-glucanes solubles dans l'eau, lesquels sont essentiellement exempts de liaisons β-(1,6), pour la préparation de compositions cosmétiques pour le maquillage selon l'une quelconque des revendications précédentes.
